# EUROPÄISCHE PATENTANMELDUNG

(11) **EP 4 674 447 A1**
(43) Veröffentlichungstag der Anmeldung: **07.01.2026**
(21) Anmeldenummer: 24185926.3
(22) Anmeldetag: 02.07.2024
(51) Int. Cl.: A61M 5/14, F16L 3/12

(54) **GEHÄUSE EINER INFUSIONSPUMPE MIT SICHERUNGSVORRICHTUNG GEGEN ENTNAHME VON EINER INFUSIONSSTANGE**

(71) Anmelder: B. Braun Melsungen AG, 34212 Melsungen (DE)
(72) Erfinder: STICH, Marcel, 34329 Nieste (DE)
(74) Vertreter: Winter, Brandl - Partnerschaft mbB

(57) **Zusammenfassung**

Offenbart ist ein abschließbares Gehäuse (2) zur zumindest abschnittsweisen Aufnahme einer Infusionspumpe (1), vorzugsweise Spritzenpumpe, wobei das Gehäuse (2) eine Klappe, insbesondere Frontklappe, (6) aufweist, die mittels einer Schließvorrichtung (22) abschließbar ist, wodurch die Klappe (6) in einer geschlossenen Position an einem Hauptabschnitt (4) des Gehäuses (2) festlegbar ist, und wobei das Gehäuse (2) mittels einer Sicherungsvorrichtung an einer Infusionsstange (12) sicherbar ist, wobei die Sicherungsvorrichtung von einer ungesicherten oder freigegebenen Position in eine gesicherte Position bewegbar ist, und wobei die Sicherungsvorrichtung in der gesicherten Position mittels der Schließvorrichtung (22) der Klappe (6) fixierbar und, insbesondere indirekt, abschließbar ist.

## Beschreibung

### Technisches Gebiet

Die vorliegende Offenbarung betrifft die Sicherung einer Infusionspumpe, z.B. Spritzenpumpe, gegen Entnahme von einer Infusionsstange oder einer Polestange oder einem Stativ.

### Hintergrund der Offenbarung

In der Medizin, z.B. auf Intensivstationen von Krankenhäusern werden Infusionspumpen, z.B. Spritzenpumpen an Infusionsstangen montiert und nach einer Therapie wieder demontiert. Die Infusionsstange wird auch Pole-Stange oder Stativ genannt. Dazu sind Klemmvorrichtungen bekannt, die einerseits an eine Rückseite eines Gehäuses der Infusionspumpe befestigt sind und anderseits an der Infusionsstange lösbar festgeklemmt werden.

### Stand der Technik

Um derartige Infusionspumpen gegen eine unbefugte Demontage und Entnahme von der Infusionsstange zu sichern, sind Kabelschlaufen bekannt, die ähnlich einem Laptopschloss sind. Diese müssen aufwändig eingefädelt werden.

Die Anmelderin selbst vertreibt ein als Lockbox bezeichnetes abschließbares Gehäuse für eine Spritzenpumpe. Das Gehäuse umfasst die betroffene Spritzenpumpe komplett bzw. haust die betroffene Spritzenpumpe komplett ein und hat eine Frontklappe, die mittels eines Schlosses abschließbar ist, womit die Frontklappe in einer geschlossenen Position an einem Hauptabschnitt des Gehäuses festgelegt wird. Derartige Gehäuse werden beispielsweise in der patientengesteuerten Schmerztherapie verwendet, bei der der Patient selbst die Menge z.B. eines Schmerzmittels bestimmen kann.

Um die Spritzenpumpe gegen eine unbefugte Demontage und Entnahme von der Infusionsstange zu sichern, kann das Gehäuse, genauer gesagt sein Hauptabschnitt, mittels einer lösbaren und abschließbaren Sicherungsvorrichtung an der Infusionsstange gesichert werden. Die Sicherungsvorrichtung hat einen Riegel, der hinter der bzw. hinter die Infusionsstange verschwenkt werden kann und mit einem Schloss abgeschlossen werden kann, so dass das Gehäuse und der Riegel die Infusionsstange allseitig umfasst.

Nachteilig an der Sicherungsvorrichtung derartiger Gehäuse ist, dass sie ein extra Schloss benötigen, womit das Gehäuse ein Schloss für die Frontklappe und ein Schloss für den Riegel der Sicherungsvorrichtung hat. Das bedeutet, dass das medizinische Personal bei der Sicherung der betroffenen Infusionspumpe zwei Schlösser bedienen muss. Dieser Nachteil vervielfacht sich entsprechend, wenn an einer Infusionsstange für einen Patienten mehrere Infusionspumpen befestigt und gesichert werden müssen.

### Kurzbeschreibung der Offenbarung

Aufgabe der vorliegenden Offenbarung ist es, ein abschließbares Gehäuse zu schaffen, das zum Einschließen einer Infusionspumpe mittels einer Klappe, beispielsweise einer Frontklappe, und mit einer abschließbaren Sicherungsvorrichtung zur Sicherung an einer Infusionsstange vorgesehen ist, wobei ein Schloss gespart werden soll bzw. entfallen soll.

Diese Aufgabe wird gelöst mit der Merkmalskombination des Anspruchs 1.

Das abschließbare Gehäuse gemäß der Offenbarung kann auch Lockbox genannt werden und ist zur zumindest abschnittsweisen Aufnahme und zum Einschließen einer Infusionspumpe, vorzugsweise Spritzenpumpe, ausgelegt und vorbereitet. Dazu hat das Gehäuse eine Klappe, insbesondere Frontklappe, die mittels einer Schließvorrichtung, insbesondere einem Schloss mit Schlüssel, abschließbar ist, wodurch die (Front-) Klappe in einer geschlossenen Position an einem Hauptabschnitt des Gehäuses festlegbar ist. Weiterhin ist der Hauptabschnitt des Gehäuses mittels einer (lösbaren und abschließbaren) Sicherungsvorrichtung an einer Infusionsstange, die auch Pole-Stange genannt werden kann, oder an einem Stativ sicherbar. Die Sicherungsvorrichtung ist von einer ungesicherten oder freigegebenen Position in eine gesicherte Position, beispielsweise translatorisch oder rotatorisch, insbesondere rotatorisch, bewegbar. Die Sicherungsvorrichtung ist in der gesicherten Position mittels der Schließvorrichtung der (Front-)Klappe fixierbar und (indirekt) abschließbar.

Die Sicherungsvorrichtung kann translatorisch von der freigegebenen zur gesicherten Position unter der Infusionspumpe nach vorne herausgezogen werden und sich dabei hinter dem Gehäuse an der Infusionsstange festlegen. Bevorzugt ist es jedoch, wenn die Sicherungsvorrichtung rotatorisch zwischen der freigegebenen Position und der gesicherten Position (bidirektional) bewegbar, insbesondere verschwenkbar ist.

Bei einem bevorzugten Ausführungsbeispiel hat die Sicherungsvorrichtung einen Hauptkörper und ein Schwenkgelenk, über das der Hauptkörper an den Hauptabschnitt des Gehäuses gekoppelt ist. Der Hauptkörper der Sicherungsvorrichtung ist mittels des Schwenkgelenks zwischen einerseits der freigegebenen Position und andererseits der gesicherten Position bidirektional verschwenkbar. Der Hauptkörper weist einen Sicherungsabschnitt (beispielsweise einen Steg) auf, der in der gesicherten Position an der (Front-)Klappe des Gehäuses festlegbar ist.

An einem bezüglich des Schwenkgelenks dem Sicherungsabschnitt gegenüberliegenden Bereich kann der Hauptkörper ein Haken aufweisen, der zum Hintergreifen einer Infusionsstange ausgelegt und eingerichtet ist. Dazu hat der Haken einen vorzugsweise bogenförmigen Anlagebereich für die Infusionsstange.

Mit einer derartigen Sicherungsvorrichtung ist es möglich, die Fixierung des betroffenen Gehäuses an der rückwärtigen Infusionsstange per Schwenkbewegung zu erreichen und gleichzeitig mit derselben Schwenkbewegung den Sicherungsabschnitt in den gegenüberliegenden vorderen Bereich der (Front-)Klappe zu bewegen, wo er fixiert werden kann.

Bei einer fertigungstechnisch einfachen Ausgestaltung ist der Hauptkörper der Sicherungsvorrichtung flächig, z.B. ein gestanztes Blechteil, an dem der Sicherungsabschnitt und gegebenenfalls auch der Haken einstückig gebildet sind. Damit kann der Hauptkörper auch als Konturhebel bezeichnet werden.

Um eine gewisse Ausdehnung des Gehäuses, genauer gesagt einen Abstand zwischen der Infusionsstange und der (Front-)Klappe zu überbrücken, wird es bevorzugt, wenn sich von dem Schwenkgelenk ein Sicherungshebel erstreckt, an dessen Endabschnitt der Sicherungsabschnitt gebildet ist, und/oder wenn der Haken über einen vorzugsweise abgewinkelten Arm mit dem Schwenkgelenk verbunden ist.

Die Sicherungsvorrichtung ist vorzugsweise über ihren Sicherungsabschnitt in der gesicherten Position vorzugsweise in einer Ausnehmung der (Front-)Klappe formschlüssig fixierbar. Damit ist der Haken im Eingriff mit der Infusionsstange fixiert und das Gehäuse ist mit der Infusionspumpe an der Infusionsstange gegen Entnahme gesichert.

Bei der Ausgestaltung der Sicherungsvorrichtung als schwenkbarer Hauptkörper ist dieser bevorzugt in der gesicherten Position mittels der Schließvorrichtung der (Front-)Klappe indirekt in folgender Weise abschließbar: Zuerst wird der Hauptkörper in die gesicherte Position verschwenkt, womit der Hauptkörper z.B. mit seinem Haken die Infusionsstange hintergreift, und gleichzeitig der Sicherungsabschnitt in den Bereich der (Front-)Klappe kommt. Dann wird die (Front-)Klappe geschlossen, womit der Sicherungsabschnitt in der Ausnehmung der (Front-)Klappe eingeführt und gegenüber der (Front-)Klappe fixiert wird. Dann wird die (Front-)Klappe mit ihrer Schließvorrichtung abgeschlossen, womit indirekt auch die Sicherungsvorrichtung fixiert und damit abgeschlossen wird.

Bei einer bevorzugten Ausgestaltung der indirekten Abschließbarkeit ist an einem ersten Rand der (Front-)Klappe ein Schwenkgelenk angeordnet, über das die (Front-) Klappe an dem Hauptabschnitt des Gehäuses angelenkt ist. Die Ausnehmung ist bevorzugt an einem dem ersten Rand gegenüberliegenden zweiten Rand der (Front-) Klappe gebildet. Die Ausnehmung ist weiter bevorzugt über oder auf den Sicherungsabschnitt bewegbar, wenn die (Front-)Klappe in ihre geschlossene Position geschwenkt wird.

In einer Einbaulage des Gehäuses mit der Infusionspumpe an der Infusionsstange ist der erste Rand bevorzugt ein oberer Rand, während der zweite Rand ein unterer Rand ist. Die Ausnehmung ist über oder auf den Sicherungsabschnitt bewegbar, wenn die (Front-)Klappe in ihre geschlossene Position heruntergeklappt wird.

Wenn der Hauptkörper flächig, z.B. als gestanztes Blechteil, ausgebildet ist, wird es besonders bevorzugt, wenn der Hauptkörper über das Schwenkgelenk an einen Boden des Hauptabschnitts des Gehäuses gekoppelt ist. Dabei ist eine Schwenkachse des Schwenkgelenks senkrecht zum Hauptkörper und senkrecht zum Boden angeordnet, und der Hauptkörper und der Boden sind parallel zueinander. Dadurch kann viel Bauraum gespart werden, genauer gesagt kann das Gehäuse klein ausgelegt werden. Der Hauptkörper und der Boden sind in der Einbaulage des Gehäuses waagerecht.

Damit auch in der freigegebenen Position und insbesondere bei Entnahme des Gehäuses von der Infusionsstange der Hauptkörper und insbesondere der Haken nicht unkontrolliert herumschwingen kann, ist vorzugsweise eine am Boden angeordnete Rastvorrichtung für den Sicherungsabschnitt vorgesehen.

Wenn zwischen der gesicherten Position und der freigegebenen Position des Hauptkörpers ein Schwenkwinkel von 45 Grad bis 135 Grad, vorzugsweise von etwa 90 Grad gebildet ist, kann der Arm in der freigegebenen Position platzsparend und der Haken möglichst nah am Gehäuse angeordnet (geparkt) werden.

Vorzugsweise erstreckt sich in der gesicherten Position des Hauptkörpers dessen Sicherungshebel, an dessen Endabschnitt der Sicherungsabschnitt gebildet ist, radial in Richtung weg vom Schwenkgelenk hin zur Ausnehmung der (Front-)Klappe.

Vorzugsweise erstreckt sich in der freigegebenen Position des Hauptkörpers dessen Sicherungshebel radial in Richtung weg vom Schwenkgelenk hin zur Rastvorrichtung, wobei der Sicherungsabschnitt in die Rastvorrichtung eingreift.

Gemäß dem Stand der Technik ist die Infusionspumpe in dem Gehäuse aufgenommen, und das Gehäuse ist mittels einer lösbaren Befestigungsvorrichtung an der Infusionsstange festlegbar. Damit ist die Infusionspumpe indirekt an der Infusionsstange festlegbar.

Demgegenüber ist mit dieser Schrift auch eine Infusionspumpenanordung mit einer Infusionspumpe offenbart, die oder deren Gehäuse mittels einer lösbaren Befestigungsvorrichtung direkt an einer Infusionsstange festlegbar, vorzugsweise mittels einer Klemmvorrichtung klemmbar ist. Dabei ist die Infusionspumpe zumindest abschnittsweise in einem Gehäuse gemäß der Offenbarung aufgenommen. Die Anmelderin behält sich vor, auf diese Infusionspumpenanordung einen Anspruch zu richten.

Damit trägt die Infusionspumpe das Gehäuse gemäß der Offenbarung, wobei das Gehäuse die Sicherungsvorrichtung trägt, die wiederum in ihrer gesicherten Position direkt die Infusionsstange hintergreift.

In dem Zusammenhang mit der Infusionspumpenanordnung mit Infusionspumpe und mit daran befestigter Klemmvorrichtung und mit dem von der Infusionspumpe getragenem Gehäuse ist die Infusionspumpe besonders gut gegen Entnahme gesichert, wenn die Klemmvorrichtung zwei Klemmbacken hat, die radial aufeinander zu bewegt werden können, und wenn der Haken der Befestigungsvorrichtung in der gesicherten Position die Infusionsstange an einer Seite hintergreift, die dem Gehäuse gegenüberliegt bzw. die vom Gehäuse abgewandt ist. In anderen Worten ausgedrückt ist die Infusionspumpe besonders gut gegen Entnahme gesichert, wenn in Umfangsrichtung der Infusionsstange betrachtet erst eine der Klemmbacken, dann der Anlagebereich des Hakens, dann die andere Klemmbacke und dann die Rückseite der Infusionspumpe bzw. des Gehäuses angeordnet sind. Mit dieser Ausgestaltung kann auch nach einem Öffnen der Klemmvorrichtung, also nach einem Auseinanderbewegen der beiden Klemmbacken, das Gehäuse mit der Infusionspumpe nicht von der Infusionsstange abgezogen werden.

### Kurzbeschreibung der Figuren

Figur 1 ist eine Infusionspumpenanordnung mit einer Infusionspumpe und mit einem Gehäuse gemäß einem Ausführungsbeispiel der vorliegenden Offenbarung in einer perspektivischen Ansicht von unten;
Figur 2 ist die Infusionspumpenanordnung aus Figur 1 in einer Ansicht von unten, wobei der Boden des Gehäuses wegelassen wurde;
Figur 3 ist eine weitere Darstellung der Infusionspumpenanordnung aus Figur 1 in einer Ansicht von unten, wobei der Boden des Gehäuses wegelassen wurde; und
Figur 4 ist eine weitere Darstellung des Ausführungsbeispiels der Infusionspumpenanordnung aus Figur 1 in einer perspektivischen Ansicht von unten.

### Beschreibung des Ausführungsbeispiels

Nachstehend wird ein Ausführungsbeispiel der vorliegenden Offenbarung auf der Basis der zugehörigen Figuren beschrieben.

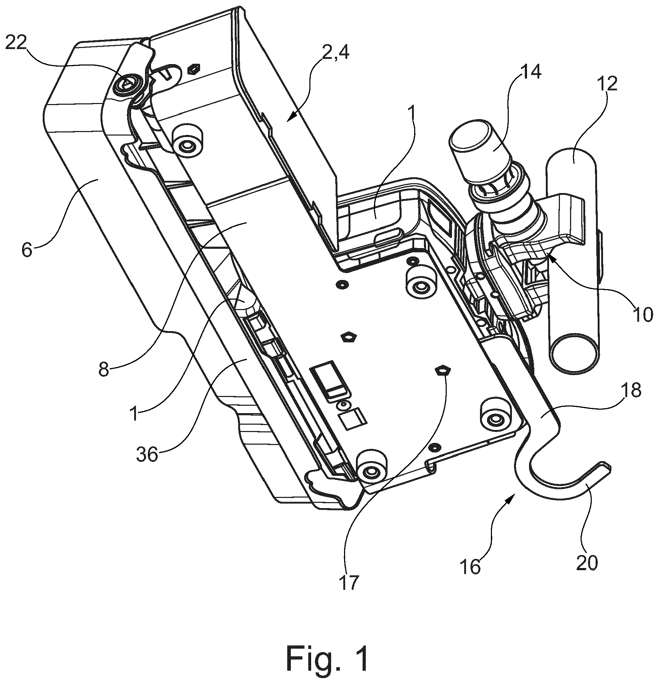

Figur 1 zeigt eine Infusionspumpenanordnung mit einer Infusionspumpe 1 und mit einem Gehäuse 2. Das Gehäuse 2 hat einen Hauptabschnitt 4 und eine dem gegenüber klappbare und schwenkbare Frontklappe 6. Dazu ist die Frontklappe 6 über ein an ihren ersten (oberen nicht sichtbaren) Rand an dem Hauptabschnitt 4 angelenkt. Von dem Hauptabschnitt 4 ist insbesondere der Boden 8 zu sehen.

Die Infusionspumpe 1 ist beim gezeigten Ausführungsbeispiel eine Spritzenpumpe, die über eine als Klemmvorrichtung ausgebildete Befestigungsvorrichtung 10 unmittelbar an einer neben einem Patientenbett stehende nur abschnittsweise gezeigte Infusionsstange 12 festgeklemmt ist. Dazu hat das Gehäuse 2 einen hinteren wandfreien Bereich, durch den hindurch sich die Befestigungsvorrichtung 10 erstreckt. Die Befestigungsvorrichtung 10 hat zwei Klemmbacken, die mittels eines drehbaren Handgriffs 14 gegeneinander gespannt sind und die Infusionsstange 12 zwischen sich einspannen.

Das Gehäuse 2 wird von der Infusionspumpe 1 getragen und dient dazu, die Infusionspumpe 1 gegen unerlaubte Manipulation und gegen unerlaubte Entnahme zu schützen. Dazu ist das Gehäuse 2 an der (in Figur 1 linken) Vorderseite, an der die Bedienteile und die Spritze angeordnet sind, mit der Frontklappe 6 komplett oder abschnittsweise schließbar. Dazu wird die Frontklappe 6 aus der in Figur 1 gezeigten halb geöffneten Zwischenposition ganz heruntergeklappt.

Weiterhin ist in Figur 1 ein Schloss 22 für einen (nicht gezeigten) Schlüssel dargestellt, das an einem seitlichen Rand des Frontklappe 6 angeordnet ist, und das in Eingriff mit dem Rand des Hauptabschnitts 4 des Gehäuses 2 gebracht werden kann. Die Besonderheit dieses Schlosses 22 wird weiter unten genauer erläutert.

Eine Sicherungsvorrichtung gemäß der Offenbarung besteht aus einem Schwenkgelenk 17 und einem flächigen Hauptkörper 16. Der Hauptkörper 16 ist in Figur 1 in seiner ungesicherten und freigegebenen Position gezeigt, in der er das Gehäuse und damit die Infusionspumpe 1 freigibt. Dabei ist der Hauptkörper 16 teilweise zwischen einerseits der Infusionspumpe 1 und andererseits dem Boden 8 des Hauptabschnitts 4 und teilweise außerhalb davon angeordnet.

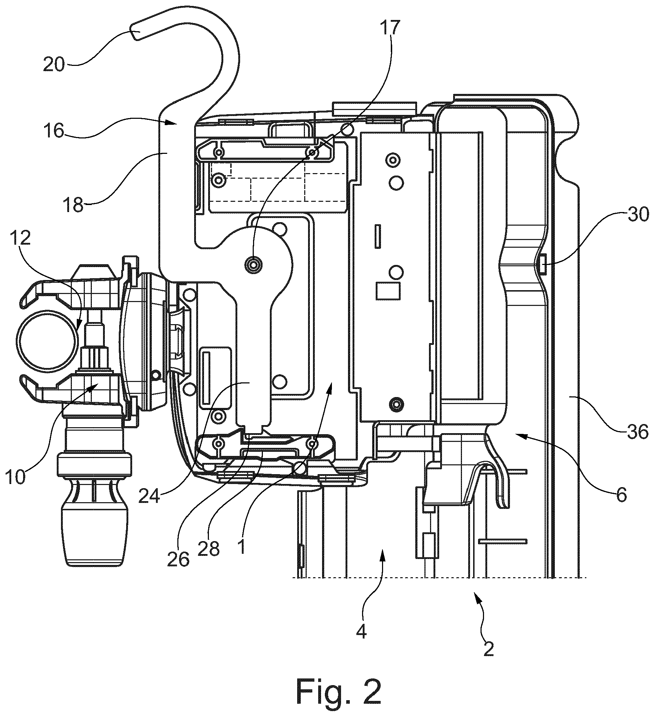

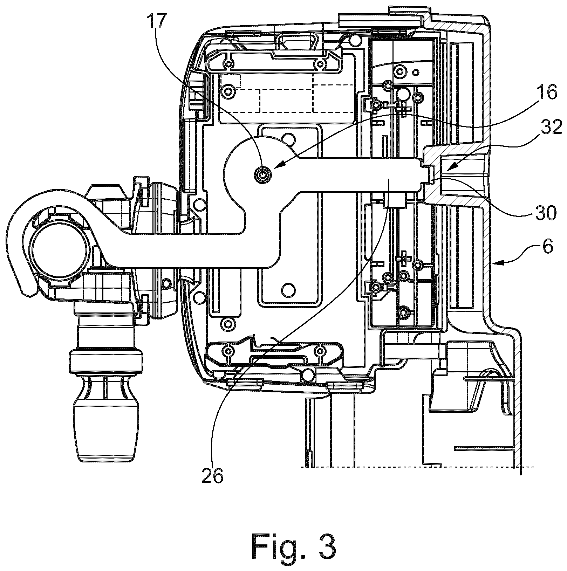

Genauer gesagt ist in der in Figur 1 gezeigten freigegebenen Position der Sicherungsvorrichtung bzw. ihres Hauptkörpers 16 ein (in den Figuren 2 und 3 gezeigter) Sicherungshebel mit einem Sicherungsabschnitt komplett zwischen dem Boden 8 des Hauptabschnitts 4 und der Infusionspumpe 1 angeordnet, während ein Arm 18 mit einem Haken 20 in seiner von der Infusionsstange 12 beabstandeten Parkposition angeordnet und daher sichtbar ist.

Figur 2 ist ein Ausschnitt des Ausführungsbeispiels der Infusionspumpenanordnung aus Figur 1 in einer Ansicht von unten, wobei der Boden 8 des Hauptabschnitts 4 des Gehäuses 2 weggelassen wurde. Abweichend von Figur 1 ist die Frontklappe 6 in ihrer ganz geöffneten Position gezeigt. In Übereinstimmung mit Figur 1 ist die Sicherungsvorrichtung bzw. ihr Hauptkörper 16 in der freigegebenen Position gezeigt.

Dabei ist zu erkennen, dass der Arm 18, der sich vom Schwenkgelenk 17 zum Haken 20 erstreckt, abgewinkelt ist. Insbesondere ist zu erkennen, dass sich vom Schwenkgelenk 17 ausgehend in einem dem Arm 18 mit dem Haken 20 gegenüberliegenden Bereich ein Sicherungshebel 24 radial erstreckt, an dessen dem Schwenkgelenk 17 abgewandten freien Endabschnitt ein Sicherungsabschnitt 26 gebildet ist.

Die beiden Klemmbacken können radial aufeinander zu bewegt werden. Der Haken 20 hintergreift in der gezeigten gesicherten Position die Infusionsstange 12 an einer (in Figur 2 linken) Seite, die dem Gehäuse 2 gegenüberliegt. Die Infusionspumpe 1 ist auch bei geöffneter Klemmvorrichtung 14 besonders gut gegen unbefugte Entnahme gesichert, da am Umfang der Infusionsstange 12 erst eine der Klemmbacken, dann der Haken 20, dann die andere Klemmbacke und dann die Rückseite der Infusionspumpe 1 bzw. des Gehäuses 2 angeordnet sind.

Der Hauptabschnitt 16 mit einerseits dem abgewinkelten Arm 18 und dem Haken 20 und andererseits mit dem Sicherungshebel 24 und dem Sicherungsabschnitt 27 sind als flächiges gestanztes Blechteil ausgebildet. Dieser flächige Hauptabschnitt 16 ist parallel zum in Figur 1 gezeigten Boden 8. Das Schwenkgelenk 17 hat eine Schwenkachse die senkrecht zur Zeichenebene der Figur 2 ist.

Der Sicherungsabschnitt 26 ist bei der in Figur 2 gezeigten freigegebenen Position in eine Rastvorrichtung 28 eingerastet und dort festgelegt, sodass insbesondere der Haken 20 nicht frei herum schwingen kann, wenn zum Beispiel das Gehäuse 2 mit der Infusionspumpe 1 von einer Infusionsstange 12 zu einer anderen transportiert wird.

Um ausgehend von der in den Figuren 1 und 2 gezeigten freigegebenen Position die Sicherungsvorrichtung bzw. das Gehäuse 2 in den an der Infusionsstange 12 gesicherten Zustand zu überführen, wird der Hauptkörper 16 gegen den Uhrzeigersinn um 90° verschwenkt, sodass der Haken 20 die Infusionsstange 12 hintergreift. Dabei wird der Sicherungsabschnitt 26 in Richtung zur Frontklappe 6 verschwenkt, so dass beim darauffolgenden Herunterklappen der Frontklappe 6 eine am Rand der Frontklappe 6 gebildete Öffnung oder Ausnehmung 30 derart über den Sicherungsabschnitt 26 bewegt wird, dass dieser in die Ausnehmung 30 eintaucht und formschlüssig gehalten wird.

Figur 3 ist ein Ausschnitt des Ausführungsbeispiels der Infusionspumpenanordnung aus Figur 1 in einer Ansicht von unten, wobei wiederum der Boden 8 des Gehäuses 2 weggelassen wurde. Die Frontklappe 6 ist in ihrer geschlossenen Position und die Sicherungsvorrichtung bzw. ihr Hauptkörper 16 in der gesicherten Position gezeigt. Die Frontklappe 6 ist geschnitten dargestellt, sodass zu erkennen ist, wie der Sicherungsabschnitt 26 in die Ausnehmung 30 eingesetzt ist.

Die Ausnehmung 30 ist in einem Bereich der Frontklappe 6 gebildet, der gleichzeitig eine Fingermulde 32 für einen Finger des medizinischen Personals bildet.

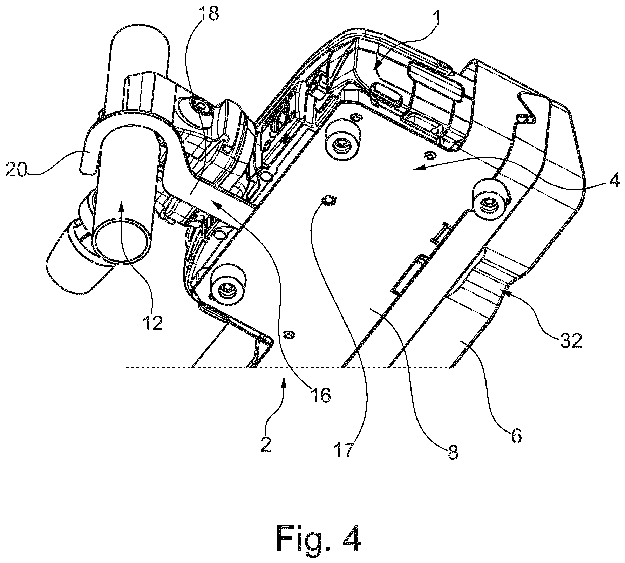

Figur 4 ist ein Ausschnitt des Ausführungsbeispiels der Infusionspumpenanordnung aus Figur 1 in einer perspektivischen Ansicht von unten. Dabei ist die Frontklappe 6 in ihrer geschlossenen Position und die Sicherungsvorrichtung bzw. ihr Hauptkörper 16 in der gesicherten Position gezeigt.

Es ist zu erkennen, dass in der gesicherten Position des Hauptkörpers 16 der Arm 18 und der Haken 20 rückseitig aus dem Gehäuse 2 hervorstehen, während der Sicherungshebel und der Sicherungsabschnitt verdeckt sind.

In der in den Figuren 3 und 4 gezeigten geschlossenen Position der Frontklappe 6 liegt der zweite (untere) Rand 36 am Hauptabschnitt 4 des Gehäuses 2 an.

In Figur 1 ist das Schloss 22 gezeigt, mit dem die Frontklappe 6 in dieser Position verriegelt wird. Dabei ist der in Figur 3 gezeigte Eingriff des Sicherungsabschnitts 32 in der Ausnehmung 30 gegeben, sodass der Hauptkörper 16 nicht um das Schwenkgelenk 17 geschwenkt werden kann und der Haken 20 in Eingriff mit der Infusionsstange 12 bleibt. Damit wird die Sicherungsvorrichtung durch das in Figur 1 gezeigte Schloss 22 indirekt gesichert. Damit ist einerseits zum Verschließen der Frontklappe 6 und andererseits zum Sichern der Infusionspumpe 1 an der Infusionsstange 12 nur ein Schloss 22 und ein (nicht gezeigter) dazu passender Schlüssel nötig.

### Bezugszeichenliste:

- 1: Infusionspumpe
- 2: Gehäuse
- 4: Hauptabschnitt
- 6: Frontklappe
- 8: Boden
- 10: Befestigungsvorrichtung / Klemmvorrichtung
- 12: Infusionsstange
- 14: Handgriff
- 16: Hauptkörper
- 18: Arm
- 20: Haken
- 22: Schließvorrichtung / Schloss
- 24: Sicherungshebel
- 26: Sicherungsabschnitt
- 28: Rastvorrichtung
- 30: Ausnehmung
- 32: Fingermulde
- 36: zweiter (unterer) Rand

## Patentansprüche

1. Abschließbares Gehäuse (2) zur zumindest abschnittsweisen Aufnahme einer Infusionspumpe (1), wobei das Gehäuse (2) eine Klappe, insbesondere Frontklappe, (6) aufweist, die mittels einer Schließvorrichtung (22) abschließbar ist, mit der die Klappe (6) in einer geschlossenen Position an einem Hauptabschnitt (4) des Gehäuses (2) festlegbar ist, und wobei das Gehäuse (2) mittels einer Sicherungsvorrichtung an einer Infusionsstange (12) sicherbar ist, wobei die Sicherungsvorrichtung von einer ungesicherten oder freigegebenen Position in eine gesicherte Position bewegbar ist, **dadurch gekennzeichnet, dass** die Sicherungsvorrichtung in der gesicherten Position mittels der Schließvorrichtung (22) der Klappe (6) fixierbar und, insbesondere indirekt, abschließbar ist.

2. Gehäuse (2) nach Anspruch 1, wobei die Sicherungsvorrichtung einen Hauptkörper (16) und ein Schwenkgelenk (17) aufweist, über das der Hauptkörper (16) an das Gehäuse (2) gekoppelt ist, und über das der Hauptkörper (16) zwischen der freigegebenen Position und der gesicherten Position bidirektional verschwenkbar ist, und wobei der Hauptkörper (16) einen Sicherungsabschnitt (26) aufweist, der an der Klappe (6) festlegbar ist.

3. Gehäuse (2) nach Anspruch 2, **dadurch gekennzeichnet, dass** an einem bezüglich des Schwenkgelenks (17) dem Sicherungsabschnitt (26) gegenüberliegenden Bereich des Hauptkörpers (16) ein Haken (20) angeordnet ist, der zum Hintergreifen der Infusionsstange (12) ausgelegt und eingerichtet ist, und der vorzugsweise über einen Arm (18) des Hauptkörpers (16) mit dem Schwenkgelenk (17) verbunden ist.

4. Gehäuse (2) nach Anspruch 2 oder 3, wobei sich von dem Schwenkgelenk (17) ein Sicherungshebel (24) des Hauptkörpers (16) erstreckt, an dessen Endabschnitt der Sicherungsabschnitt (26) gebildet ist.

5. Gehäuse (2) nach einem der Ansprüche 2 bis 4 **dadurch gekennzeichnet, dass** der Hauptkörper (16) flächig ist, und dass an dem Hauptkörper (16) der Sicherungsabschnitt (26) einstückig gebildet ist.

6. Gehäuse (2) nach einem der Ansprüche 2 bis 5 **dadurch gekennzeichnet, dass** die Sicherungsvorrichtung über ihren Sicherungsabschnitt (26) in der gesicherten Position in einer Ausnehmung (30) der Klappe (6) formschlüssig fixierbar ist.

7. Gehäuse (2) nach Anspruch 6 **dadurch gekennzeichnet, dass** an einem ersten Rand der Klappe (6) ein Schwenkgelenk angeordnet ist, über das die Klappe (6) an den Hauptabschnitt (4) des Gehäuses (2) angelenkt ist, wobei die Ausnehmung (30) im Bereich eines zweiten Randes (36) oder an einem zweiten Rand (36) der Klappe (6) gebildet ist, und wobei die Ausnehmung (30) über oder auf den Sicherungsabschnitt (26) bewegbar ist, wenn die Klappe (6) in ihre geschlossene Position geschwenkt wird.

8. Gehäuse (2) nach einem der Ansprüche 2 bis 7 **dadurch gekennzeichnet, dass** der Hauptkörper (16) flächig ist und über das Schwenkgelenk (17) an einen Boden (8) des Hauptabschnitts (4) des Gehäuses (2) gekoppelt ist, wobei eine Schwenkachse des Schwenkgelenks (17) senkrecht zum Hauptkörper (16) und senkrecht zum Boden (8) angeordnet ist, und wobei der Hauptkörper (16) und der Boden (8) parallel zueinander sind.

9. Gehäuse (2) nach Anspruch 8 **gekennzeichnet durch** eine am Boden (8) angeordneten Rastvorrichtung (28) für den Hauptkörper (16) in der freigegebenen Position.

10. Gehäuse (2) nach einem der Ansprüche 2 bis 9 **dadurch gekennzeichnet, dass** zwischen der gesicherten Position und der freigegebenen Position des Hauptkörpers (16) ein Schwenkwinkel von 45 Grad bis 135 Grad, insbesondere von etwa 90 Grad gebildet ist.

11. Gehäuse (2) nach einem der Ansprüche 2 bis 10 **dadurch gekennzeichnet, dass** sich in der gesicherten Position des Hauptkörpers (16) ein Sicherungshebel (24), an dessen Endabschnitt der Sicherungsabschnitt (26) gebildet ist, in Richtung weg vom Schwenkgelenk (17) hin zur Ausnehmung der Klappe (6) erstreckt.

12. Gehäuse (2) nach den Ansprüchen 9 und 11 **dadurch gekennzeichnet, dass** in der freigegebenen Position des Hauptkörpers (16) sich der Sicherungshebel (24) in Richtung weg vom Schwenkgelenk (17) hin zur Rastvorrichtung (28) erstreckt.
